# EUROPEAN PATENT APPLICATION

(11) **EP 3 777 779 A2**
(43) Date of publication of application: **17.02.2021**
(21) Application number: 20177694.5
(22) Date of filing: 18.04.2017
(51) Int. Cl.: A61F 2/52, A41C 3/00, A41C 3/14, A61F 5/058, A61F 2/50

(54) **STRESS RELIEVING DEVICE**

(30) Priority: 19.04.2016 US 201662324571 P
(62) Divisional of application: 17722208.0
(71) Applicant: LifeCell Corporation, Madison, New Jersey 07940 (US)
(72) Inventor: PARK, Sangwook, Dunellen, New Jersey 08812 (US); BARERE, Aaron, Hoboken, New Jersey 07030 (US); FRIEDMAN, Evan, West Orange, New Jersey 07052 (US)
(74) Representative: Forresters IP LLP

(57) **Abstract**

The present disclosure relates generally to devices and methods to relieve tension or stress on tissue and, in particular, tissue that includes a surgical incision site. A shape-setting material is used to conform the device to the tissue without the need for continuous application of negative pressure. The device can support and improve the health of breast tissue after breast augmentation or reconstruction post-mastectomy.

## Description

This application claims priority to United States Provisional Application No. 62/324,571, filed April 19, 2016, the entire contents of which is incorporated herein by reference.

The present disclosure relates generally to devices and methods to relieve tension or stress on tissue and, in particular, tissue near a surgical site.

Breast reconstruction mastectomy or breast augmentation procedures typically involve implantation of a synthetic implant such as a silicone- or saline-filled implant. In recent years, there has been a greater acceptance of skin-sparing and skin/nipple-sparing mastectomies. With such procedures, there is more skin for reconstruction and less of a need for expansion of the breast-skin envelope. Sometimes, breast skin flaps are less healthy because of diminished blood supply from underlying breast tissue removed during mastectomy. Among other things, there is not always a sufficient blood supply to clear infections or to support natural healing, and this issue can be especially challenging in the presence of synthetic implants. As a result, surgeons have found that the incidence of implant loss is reduced when implants are placed under well vascularized muscle or rapidly vascularizing acellular dermal matrix (ADM).

Vascularization of muscle, tissue, or skin relies on strong blood flow to the region. If breast tissue after surgery is unsupported or improperly supported during healing, blood flow to the tissue can be reduced. Breast implants tend to be relatively heavy, and implants placed during surgery can weigh on tissue that is undergoing recovery. Surgical outcomes can be improved if stresses on healing tissue can be reduced.

Similarly, healing of other surgical sites can be adversely affected if the site is insufficiently supported. In particular, healing of surgical incisions after reconstruction or augmentation of breast tissue, or other surgical sites, can be slowed by tension on the tissues and the surgical incision site. In addition, current systems to support breast tissue can place undue pressure or friction directly on the site of the incision, potentially leading to adverse outcomes such as infection or dehiscence.

Accordingly, the present disclosure provides improved devices to relieve stresses on tissues near an incision, as well as the incision itself, to improve surgical outcomes. The devices provide a barrier against external contamination and help to hold the incision margins together.

Disclosed herein is a device for treating an anatomical region near a surgical site. The device includes a body including an interior space. The device also includes a shape-setting material disposed within the interior space. The device further includes a port disposed on the body and in fluid communication with the interior space. Application of negative pressure through the port causes the body to conform to at least a portion of an anatomical region near a surgical site. Setting the shape-setting material causes the body to continue to conform to the portion of the anatomical region upon removal of the negative pressure through the port.

Disclosed herein is a method of relieving stress on an anatomical region near a surgical site. The method includes providing a device including a body having an interior space and a port in fluid communication with the interior space. The interior space includes a shape-setting material. The method also includes applying the device to an anatomical region near a surgical site. The method further includes applying a negative pressure through the port to cause the body to conform to at least a portion of the anatomical region. The method also includes setting the shape-setting material such that the body continues to conform to the portion of the anatomical region upon removal of the negative pressure through the port.

Disclosed herein is a method of treating breast tissue. The method includes providing a device including a body comprising one or two cups, an interior space, and a port in fluid communication with the interior space. The interior space includes a shape-setting material. The method also includes applying the device to breast tissue having at least one incision using an adhesive layer on an inner surface of the device. The method further includes applying a negative pressure through the port to cause the body to conform to at least a portion of the breast tissue. The method also includes setting the shape-setting material such that the body continues to conform to the portion of the breast tissue upon removal of the negative pressure through the port.

Disclosed herein is a method of treating a breast. The method includes performing a surgical procedure on a breast. The method also includes implanting an implant within a space within the breast. The method further includes closing a surgical incision to maintain the implant within the space within the breast. The method also includes providing a device including a body having an interior space and a port in fluid communication with the interior space. The interior space includes a shape-setting material. The method further includes applying the device to the breast near the surgical incision. The method also includes applying a negative pressure through the port to cause the body to conform to at least a portion of the breast. The method further includes setting the shape-setting material such that the body continues to conform to the breast upon removal of the negative pressure through the port.

### BRIEF DESCRIPTION OF THE DRAWINGS

Reference will now be made to exemplary embodiments, examples of which are illustrated in the accompanying drawings. Wherever possible, the same reference numbers will be used throughout the drawings to refer to the same or like parts. The drawings are not necessarily to scale.
Fig. 1 depicts a device for providing post-surgical support of a surgical site in accordance with various embodiments of the present disclosure.
Fig. 2A depicts a cross-sectional view of a device for providing post-surgical support of a surgical site applied to a tissue in accordance with various embodiments of the present disclosure.
Fig. 2B depicts a perspective view of a device for providing post-surgical support of a surgical site applied to a tissue in accordance with various embodiments of the present disclosure.
Fig. 2C depicts a cross-sectional view of a device including multiple interior spaces for providing post-surgical support of a surgical site applied to a tissue in accordance with various embodiments of the present disclosure.
Fig. 3A depicts a perspective view of a device for providing post-surgical support of a surgical site applied to a tissue in accordance with various embodiments of the present disclosure.
Fig. 3B depicts a cross-sectional view of a device for providing post-surgical support of a surgical site applied to a tissue in accordance with various embodiments of the present disclosure.
Fig. 4 depicts a method of relieving stress on a tissue having an incision in accordance with various embodiments of the present disclosure.
Fig. 5 depicts a method of treating breast tissue in accordance with various embodiments of the present disclosure.
Fig. 6 depicts a method for treating a breast in accordance with various embodiments of the present disclosure.

### DESCRIPTION OF EXEMPLARY EMBODIMENTS

Reference will now be made in detail to various embodiments of the disclosed devices and methods, examples of which are illustrated in the accompanying drawings. Wherever possible, the same reference numbers will be used throughout the drawings to refer to the same or like parts.

In this application, the use of the singular includes the plural unless specifically stated otherwise. In this application, the use of "or" means "and/or" unless stated otherwise. Furthermore, the use of the term "including," as well as other forms such as "includes" and "included", is not limiting. Any range described herein will be understood to include the endpoints and all values between the endpoints. The term "fluid" will be understood in this application to refer to and include both liquids and gases. The term "incision" will be understood in this application to refer to and include both iatrogenic surgical incisions and wounds sustained under other conditions. In this application, "setting the shape-setting material" will be understood to mean causing the shape-setting material to set through any means, or, in the case of a self-setting material, will be understood to mean allowing the material to set or creating conditions that allow the material to set.

The section headings used herein are for organizational purposes only and are not to be construed as limiting the subject matter described. All documents, or portions of documents, cited in this application, including but not limited to patents, patent applications, articles, books, and treatises, are hereby expressly incorporated by reference in their entirety for any purpose.

The present disclosure relates to devices and methods to relieve stress or tension around surgical sites. The devices are capable of conforming to a tissue when a negative pressure is applied to the device. The devices can also include a shape-setting material. After setting of the shape-setting material, the device can continue to conform to the tissue after removal of the negative pressure. The device can provide a mechanical barrier against external contamination, help to hold incision margins together, and enhance blood flow throughout supported tissue.

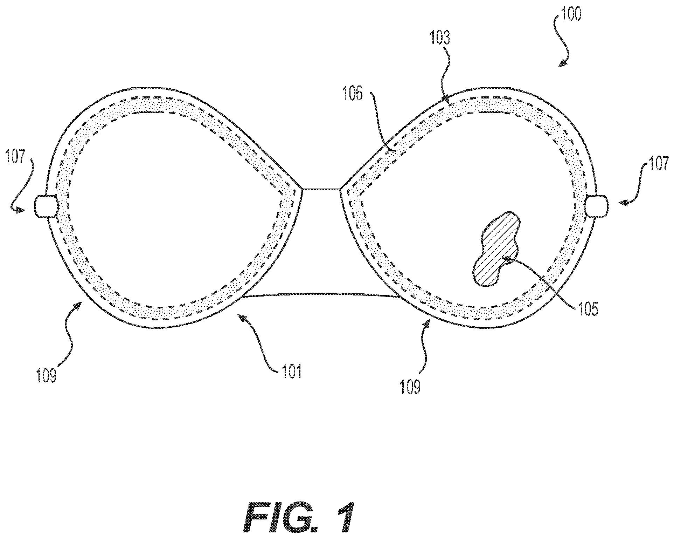

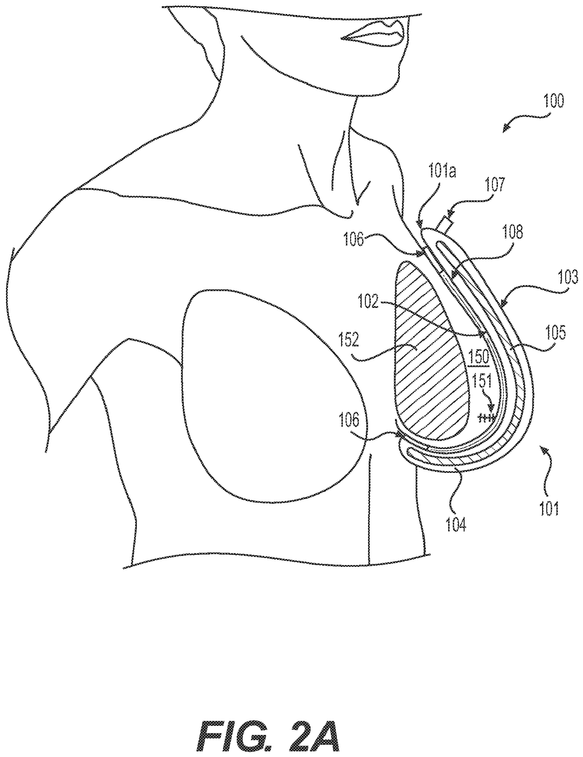

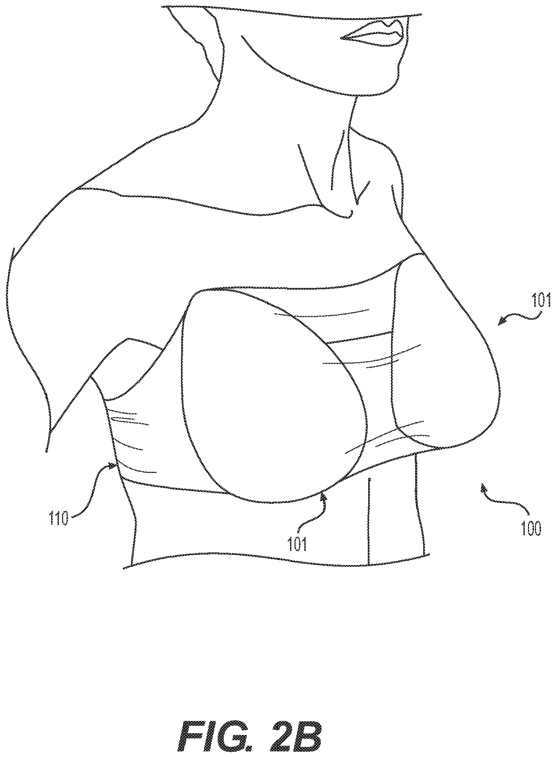

Fig. 1 depicts a device 100 for post-surgical application according to various embodiments of the present disclosure. The device 100 can relieve stress or tension on tissues including tissues near or abutting an incision. The device 100 is depicted in Figs. 2A and 2B applied to a surgical site 150 having an incision 151 and, in some embodiments, an implant 152. The device 100 can include a body 101 having an inner layer 102 and an outer layer 103. An interior space 104 can be disposed between the inner layer 102 and the outer layer 103. The interior space 104 can include a shape-setting material 105. A port 107 in fluid communication with the interior space 104 can be disposed on the body 101. By setting the shape-setting material 105 in the interior space 104, the device 100 can be fixed in a conformation to provide mechanical support to the surgical site 150 without the need for continued application of negative pressure through the port 107.

The body 101 of the device 100 can be flexible to conform to at least a portion of the tissue for which the stress is to be relieved. The body 101 can be made of a variety of suitable materials. In some embodiments, the body 101 can be formed of a single sheet of material that is folded to create the inner layer 102 and the outer layer 103. In other embodiments, the body 101 can be formed of an initially separated inner layer 102 and outer layer 103 of material that are joined at a seam 101a to form the body 101. The seam 101a can be formed by use of adhesives or glues, heat-sealing, crimping together of the layers, or any other suitable method. In an exemplary embodiment, the body 101 of the device 100 can be waterproof or water-resistant to keep the incision 151 protected from moisture while, for example, the wearer showers. In some embodiments, the device 100 can include one or two separated cup-like portions 109 that may be linked by connecting elements as in, for example, a brassiere. The cup-like portions 109 can be sized and shaped to cover some or all of the breast tissue to provide support to the breast tissue.

As shown in Fig. 2A, the inner layer 102 can contact the tissue 150 of a wearer. In various embodiments, the inner layer 102 and the outer layer 103 can be formed of the same or different materials. The material composition of the inner layer 102 or outer layer 103 can include polymers, film dressings, silicone, or any other material that is compatible with skin contact. In some embodiments, the materials of the inner layer 102 or outer layer 103 can be breathable, waterproof, transparent, or sterilizable. In some embodiments, the inner layer 102 and the outer layer 103 can use materials similar to those in the V.A.C. ® Therapy units (KCI Licensing, San Antonio, TX). In an exemplary embodiment, the outer layer 103 can be formed of a material that is puncture-resistant or leak-proof to prevent air from entering the body 101 or interior volume 104 during application of negative pressure.

To secure the device 100 to the tissue 150 during initial placement and during subsequent use, an inner surface of the inner layer 102 can include an adhesive layer 106 made of a tacky, sticky, or adherent material. In some embodiments, the adhesive layer does not contact the incision 151. The adhesive layer can include, but is not limited to, tapes, fibrin sealants, cyanoacrylates and other acrylate polymers, hydroabietic acid, gelatin and thrombin products, polyethylene glycol polymers, albumin and glutaraldehyde products, and any other suitable adhesive element. In some embodiments, the adhesive layer 106 can attach to the user at least on or below the breast and on or above the breast to provide support for the breast. The adhesive layer 106 can be formed as an unbroken surface on the inner surface of the inner layer 102 or can be formed in segments. In some embodiments, the interior volume 104 can include holes or passages through to the inner surface of the inner layer 102 so that the surface of the tissue 150 and the interior volume are in fluid communication. In such an embodiment, the adhesive layer 106 can provide an airtight barrier to allow vacuum to be applied near the tissue 150 through the interior volume 104. Application of vacuum through the interior volume 104 to the vicinity of the tissue 150 can facilitate removal of accumulating fluids from the incision 151.

Additional or alternative methods of holding the device 100 to a tissue 150 are contemplated. In some embodiments, the device 100 can additionally or alternatively be held in place on the tissue 150 using a drape 110 (Fig. 2B). The drape 110 can cover at least a portion of the device 100 and prevent the device 100 from separating from the tissue 150. In some embodiments, the drape 110 can be waterproof or water-resistant to protect the device 100 and underlying tissue 150 from coming into contact with water. In some embodiments, the drape 110 can be an elastic bandage such as an ACE™ elastic bandage (3M Corporation, St. Paul, MN) or can include conventional brassiere mechanisms such as straps. In some embodiments, the drape 110 can include TEGADERM™ (3M Corporation, St. Paul, MN) or other adhesive films for wound dressings. In such embodiments, the drape 110 may be attached both to the outer layer 103 or any other suitable element of the device 100 and to tissue surrounding the tissue 150 having an incision 151. In some embodiments, the drape 110 can provide an airtight barrier to allow vacuum to be applied near the tissue 150 and within the interior volume 104. In such an embodiment, the inner layer 102 can be partially complete (i.e., the inner layer 102 can have holes).

In some embodiments, the device 100 can include a wicking layer 108 to draw fluid away from the incision 151 and, optionally, towards the port 107 for removal. The wicking layer 108 can include antimicrobial agents. Any suitable antimicrobial agent can be used by application or implantation into the wicking layer 108 or other surface of the device 100 of the present disclosure. In certain embodiments, the antimicrobial agent includes at least one of benzethonium chloride, chlorhexidine, benzalkonium chloride, and silver nitrate. In other embodiments, the antimicrobial agent includes poly-hexa-methylene biguanide hydrochloride, silver ions, or chitosan. In some embodiments, suitable antimicrobial compounds also include cationic peptides such as nisin, cathelicidin, and abaecin. Additional suitable anti-microbial compounds include quaternary ammonium compounds such as methylbenzethonium chloride, cetalkonium chloride, dofanium chloride, tetraethylammonium bromide, didecyldimethylammonium chloride, and domiphen bromide. In some embodiments, combinations of different anti-microbial agents can be used. Examples of antimicrobial combinations include silver and chlorhexidine as well as chlorhexidine with various quaternary ammonium compounds.

Antibiotics or other therapeutic compounds may be applied to surfaces that contact the tissue such as the wicking layer 108, the adhesive layer 106, or the inner layer 102. Exemplary therapeutic compounds can include antibiotics such as penicillin, gentamycin, or streptomycin; anti-inflammatory compounds such as non-steroidal anti-inflammatory drugs; numbing agents; or other formulations as needed for a specific application.

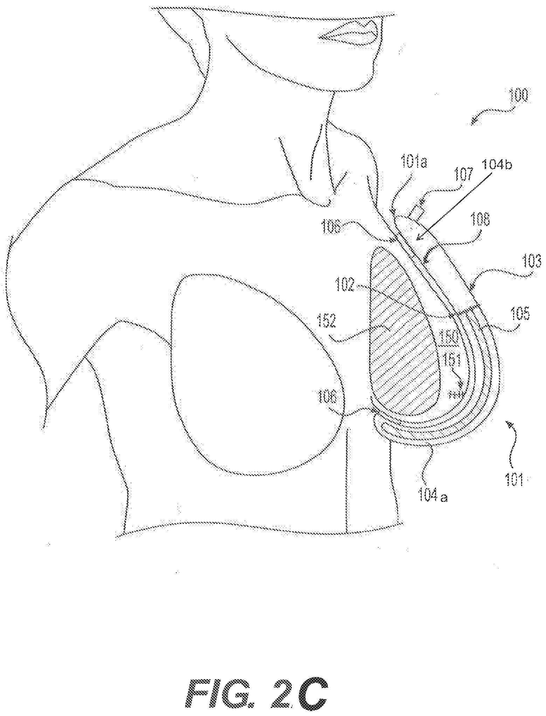

In some embodiments, the device 100 can include a first interior space 104a and a second interior space 104b that is separate from the first interior space 104a as shown in Fig. 2C. The first interior space 104a and the second interior space 104b can be isolated from one another or can be in fluid communication through a small channel. In some embodiments, the shape-setting material 105 can be placed in the first interior space 104a and the port 107 can be in fluid communication with the second interior space 104b. In this way, the incision site can be in fluid communication with one interior space 104b and a vacuum pump, and the shape-setting material 105 can be isolated in a different interior space 104a. The first interior space 104a and the second interior space 104b can have different ports that can be individually opened and closed. Separation of the first interior space 104a from the second interior space 104b can enable the use of non-porous materials as the shape-setting material 105. The first and second interior spaces 104a, 104b can be arranged to correspond to different locations on or around the tissue 150. In some embodiments, the first and second interior spaces 104a, 104b can correspond to different quadrants of the tissue 150, the upper pole and lower pole of the breast, the incision site and a spared site, the breast and a torso, or any other suitable positioning scheme. In some embodiments, the first interior space 104a and the second interior space 104b are arranged in layers of the device 100.

The shape-setting material 105 can initially be in an expanded state and can transform to a compressed state upon application of negative pressure. In some embodiments, the shape-setting material 105 can include a foam or a sponge-like material. As a non-limiting example, the shape-setting material 105 can include a foam such as GranuFoam™ as found in Prevena™ dressings (KCI, San Antonio, TX). Collapse or compression of the shape-setting material 105 under vacuum can allow the shape-setting material 105 to closely conform to the tissue 150 to which the device 100 is applied.

Mechanical support can relieve stress on the tissue 150 particularly in surgical indications where a relatively heavy implant 152 has been used. In some embodiments, the shape-setting material 105 can be a self-setting polymer or foam, cement, rubber cement, cyanoacrylate, dental putty or caulk, or two-part epoxy. In shape-setting material 105 can be a UV-curing liquid silicone rubber or a thermoset polymer such as polymethyl methacrylate (PMMA). In some embodiments, the shape-setting material 105 can be a pre-formed polyurethane foam including, for example, PMMA or a biocompatible cement. The foam can undergo compression and can conform to an anatomical feature when placed under vacuum at which point the PMMA or cement can be cured to lock in the shape. The shape-setting material 105 can harden in a manner similar to, for example, a cast to provide mechanical support to the tissue 150. In various embodiments, application of the device 100 to the tissue or setting of the shape-setting material 105 can occur while the user is positioned upright or supine.

In accordance with various embodiments, the shape-setting material 105 can be self-setting or can be activated through an external stimulus such as, but not limited to, ultraviolet light, heat, or the activation of a reactive component to the shape-setting material 105. In some embodiments, the port 107 can be used to introduce the reactive component (e.g., a hardener, an activating solution, or one of the two parts of a two-part epoxy) into the interior space 104 before the application of negative pressure. In some embodiments, the process of setting the shape-setting material 105 is insufficiently exothermic to cause discomfort to a wearer or to damage the tissue 150. In some embodiments, the device 100 can include a heat-shielding or heat-reflecting material that thermally insulates the tissue 150 from the shape-setting material 105. In some embodiments, the shape-setting material 105 can have a filamentous, granular, or porous structure to improve the evacuation of air from the interior space 104 when a negative pressure is applied at the port 107.

The port 107 can allow the introduction or extraction of fluids from the interior space 104 of the device 100. The port 107 can include various fittings to facilitate connection of gas or liquid sources or vacuum sources. The fittings can include Luer fittings or locks, inflation valves or stems, threads, or any other suitable connection or coupling mechanism. In some embodiments, the port 107 can include a one-way valve that only allows extraction of fluids. In some embodiments, one or more ports 107 can extend through the inner layer and can be used to remove accumulated fluid from the incision 151. In other embodiments, the inner layer 102 can have a hole or holes to allow fluids to pass into the interior volume 104 where they are eventually evacuated through one or more ports 107. In certain embodiments, the port 107 can be sealed temporarily or permanently to prevent further transfer of fluids into or out of the interior volume 104. In one embodiment, the interior volume 104, the first interior volume 104a, or the second interior volume 104b can be further subdivided to provide multiple chambers that, for example, can be evacuated independently through independent ports 107 or can contain separate portions of shape-setting material 105 that can be set at different times. In some embodiments, applying a vacuum source to the port 107 can cause the adhesive layer 106 to firmly contact the tissue thereby improving the adhesion.

The device 100 can be designed to fit the body shape or tissue shape of the user. In some embodiments, the device 100 or portions of the device can be sized according to standard women's brassiere sizing conventions. The device 100 can be worn for various lengths of time as required by a user or by a treatment regimen. For example, the device 100 can be worn between dressing changes during recovery from surgery at which time an old device may be replaced with a new device. Periodic replacement of the device 100 can continue until the incision or tissue has healed or until another recovery milestone is reached such as removal of surgical drains from a user. In some embodiments, the device 100 can be worn for one day, several days, one week, or any time period in between as needed for a specific application.

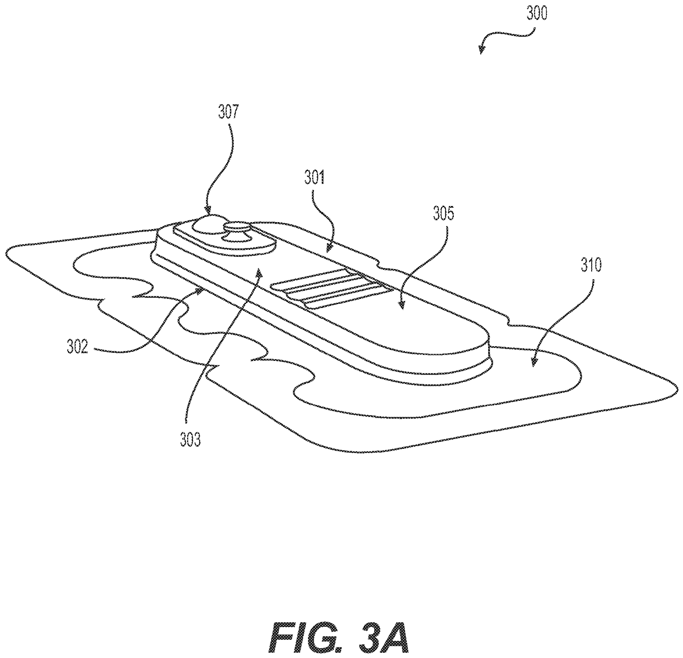

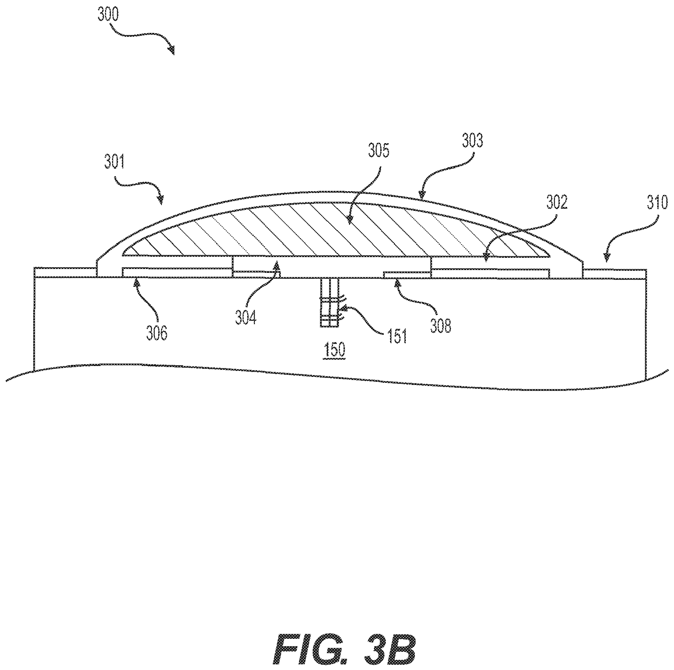

Figs. 3A and 3B depict a device 300 for post-surgical application in accordance with the present disclosure. Fig. 3A depicts a perspective view, while Fig. 3B depicts a cross-sectional view of the device 300 applied to tissue 150 near an incision 151. The device 300 can relieve stress or tension on tissues including tissues near or abutting an incision. The device 300 can include a body 301 having an inner layer 302 and an outer layer 303. An interior space 304 can be disposed between the inner layer 302 and the outer layer 303. The interior space 304 can include a shape-setting material 305. A port 307 in fluid communication with the interior space 304 can be disposed on the body 301. By setting the shape-setting material 305 in the interior space 304, the device 300 can be fixed in a conformation to provide mechanical support to the tissue 150 without the need for continued application of negative pressure through the port 307.

The body 301 of the device 300 can be flexible to conform to at least a portion of the tissue for which the stress is to be relieved. The body 301 can be made of a variety of suitable materials. In some embodiments, the body 301 can be formed of a single sheet of material that is folded to create the inner layer 302 and the outer layer 303. In other embodiments, the body 301 can be formed of an initially separated inner layer 302 and outer layer 303 of material. The inner layer 302 and outer layer 303 can be joined at a seam or can press together upon application of negative pressure. In an exemplary embodiment, the body 301 of the device 300 can be waterproof or water-resistant to keep an incision protected from moisture while, for example, the wearer showers. In some embodiments, the body 301 can be formed to have a shape that conforms to tissue to be treated. For example, the body 301 can have a shape similar to a belly sling or girdle to treat certain abdominal wounds.

As shown in Fig. 3B, the inner layer 302 can contact tissue of a wearer. In various embodiments, the inner layer 302 and the outer layer 303 can be formed of the same or different materials. The material composition of the inner layer 302 or outer layer 303 can include polymers or any other suitable material. In some embodiments, the materials of the inner layer 302 or outer layer 303 can be sterilizable. In an exemplary embodiment, the outer layer 303 can be formed of a material that is puncture-resistant or leak-proof to prevent air from entering the body 301 or interior volume 304 during application of negative pressure.

To better adhere the device 300 to the tissue during initial placement and during subsequent wear, the inner layer 302 can include an adhesive layer 306 made of a tacky, sticky, or adherent material. In some embodiments, the adhesive layer does not contact the incision. The adhesive layer can include, but is not limited to, tapes, fibrin sealants, cyanoacrylates and other acrylate polymers, hydroabietic acid, gelatin and thrombin products, polyethylene glycol polymers, albumin and glutaraldehyde products, and any other suitable adhesive element. The adhesive layer 306 can be formed as an unbroken surface on the inner layer 302 or can be formed in segments. In some embodiments, the adhesive layer 306 can provide an airtight barrier to allow vacuum to be applied near the tissue and within the interior volume 304. In such an embodiment, the inner layer 302 can be partially complete (i.e., the inner layer 302 can have holes).

Additional or alternative methods of holding the device 300 fast to a tissue 150 are contemplated. In some embodiments, the device 300 can additionally or alternatively be held in place on the tissue using a drape 310. The drape 310 can cover at least a portion of the device 300 and prevent the device 300 from separating from the tissue. In some embodiments, the drape 310 can be waterproof or water-resistant to protect the device 300 and underlying tissue from coming into contact with water. In some embodiments, the drape 310 can be an elastic bandage such as an ACE™ elastic bandage (3M Corporation, St. Paul, MN). In some embodiments, the drape 310 can include TEGADERM™ (3M Corporation, St. Paul, MN) or other adhesive films for wound dressings. In such embodiments, the drape 310 may be attached both to the outer layer 303 or any other suitable element of the device 300 and to tissue surrounding the tissue having an incision. In some embodiments, the drape 310 can provide an airtight barrier to allow vacuum to be applied near the tissue and within the interior volume 304. In such an embodiment, the inner layer 302 can be partially complete (i.e., the inner layer 302 can have holes).

In some embodiments, the device 300 can include a wicking layer 308 to draw accumulating fluid away from the incision and to the port 307 for removal. The wicking layer 308 can include antimicrobial agents. Any suitable antimicrobial agent can be used by application or implantation into the wicking layer 308 or other surface of the device 300 of the present disclosure. In certain embodiments, the antimicrobial agent includes at least one of benzethonium chloride, chlorhexidine, benzalkonium chloride, and silver nitrate. In other embodiments, the antimicrobial agent includes poly-hexa-methylene biguanide hydrochloride, silver ions, or chitosan. In some embodiments, suitable antimicrobial compounds also include cationic peptides such as nisin, cathelicidin, and abaecin. Additional suitable anti-microbial compounds include quaternary ammonium compounds such as methylbenzethonium chloride, cetalkonium chloride, dofanium chloride, tetraethylammonium bromide, didecyldimethylammonium chloride, and domiphen bromide. In some embodiments, combinations of different anti-microbial agents can be used. Examples of antimicrobial combinations include silver and chlorhexidine as well as chlorhexidine with various quaternary ammonium compounds.

Antibiotics or other therapeutic compounds may be applied to surfaces that contact the tissue such as the wicking layer 308, the adhesive layer 306, or the inner layer 302. Exemplary therapeutic compounds can include antibiotics such as penicillin, gentamycin, or streptomycin; anti-inflammatory compounds such as non-steroidal anti-inflammatory drugs; numbing agents; or other formulations as needed for a specific application.

The shape-setting material 305 can initially be in an expanded state and can transform to a compressed state upon application of negative pressure. In some embodiments, the shape-setting material 305 can include a foam or a sponge-like material. As a non-limiting example, the shape-setting material 305 can include a foam such as GranuFoam™ as found in Prevena™ dressings (KCI, San Antonio, TX). Collapse or compression of the shape-setting material 305 under vacuum can allow the shape-setting material 305 to closely conform to tissue to which the device 300 is applied.

In some embodiments, the shape-setting material 305 can be a self-setting polymer or foam. The shape-setting material 305 can advantageously harden in a manner similar to, for example, a cast to provide mechanical support to the tissue. In various embodiments, application of the device 300 to the tissue or setting of the shape-setting material 305 can occur while the user is positioned upright or supine.

In accordance with various embodiments, the shape-setting material 305 can be self-setting or can be activated through an external stimulus such as, but not limited to, ultraviolet light, heat, or the activation of a reactive component to the shape-setting material 305. In some embodiments, the port 307 can be used to introduce the reactive component (e.g., a hardener, an activating solution, or one of the two parts of a two-part epoxy) into the interior space 304 before the application of negative pressure. In some embodiments, the process of setting the shape-setting material 305 is insufficiently exothermic to cause discomfort to a wearer or to damage the tissue. In some embodiments, the device 300 can include a heat-shielding or heat-reflecting material that thermally isolates the tissue from the shape-setting material 305. In some embodiments, the shape-setting material 305 can have a filamentous, granular, or porous structure to improve the evacuation of air from the interior space 304 when a negative pressure is applied at the port 307.

The port 307 can allow the introduction or extraction of fluids from the interior space 304 of the device 300. The port 307 can include various fittings to facilitate connection of gas or liquid sources or vacuum sources. The fittings can include Luer fittings or locks, inflation valves or stems, threads, or any other suitable connection or coupling mechanism. In some embodiments, the port 307 can include a one-way valve that only allows extraction of fluids. In some embodiments, one or more ports 307 can extend through the inner layer and can be used to remove fluid from an incision. In other embodiments, the inner layer 302 can have a hole or holes to allow fluids to pass into the interior volume 304 where they are eventually evacuated through one or more ports 307. In certain embodiments, the port 307 can be sealed temporarily or permanently to prevent further transfer of fluids into or out of the interior volume 304. In any embodiment, the interior volume 304 can be further subdivided to provide multiple chambers that, for example, can be evacuated independently through independent ports 307 or can contain separate portions of shape-setting material 305 that can be set at different times. In some embodiments, applying a vacuum source to the port 307 can cause the adhesive layer 306 to firmly contact the tissue thereby improving the adhesion.

The device 300 can be designed to fit the body shape or tissue shape of the user. The device 300 can be worn for various lengths of time as required by a user or by a treatment regimen. For example, the device 300 can be worn between dressing changes during recovery from surgery at which time the old device 300 may be replaced with a new device 300. Periodic replacement of the device 300 can continue until the incision or tissue has healed or until another recovery milestone is reached such as removal of surgical drains from a user. In some embodiments, the device 300 can be worn for one day, several days, one week, or any time period in between as needed for a specific application.

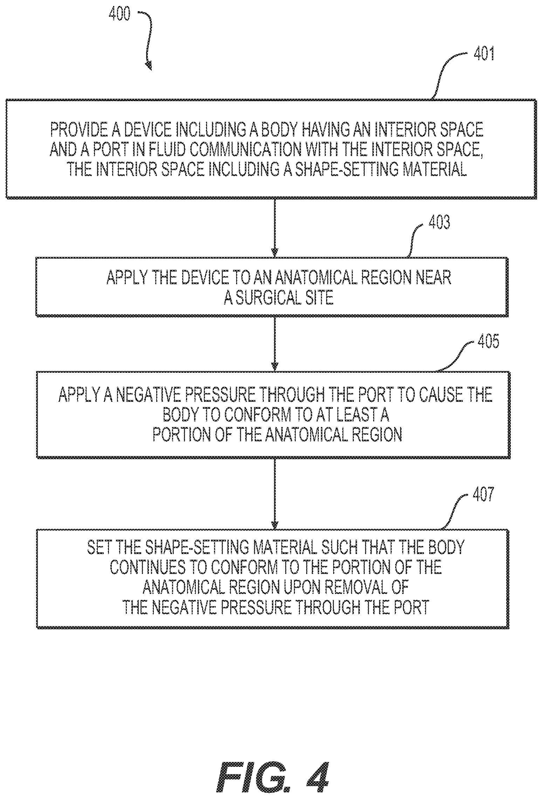

As shown in Fig. 4, a method 400 of relieving stress on an anatomical region near a surgical site is provided in accordance with various embodiments of the present disclosure. Although the method is described with respect to specific devices and figures, it will be understood that the methods can be implemented using any of the various embodiments discussed herein unless otherwise inconsistent with the description. The method 400 includes providing a device including a body having an interior space and a port in fluid communication with the interior space (step 401). The interior space includes a shape-setting material. The method 400 also includes applying the device to a tissue having an incision (step 403). The method 400 further includes applying a negative pressure through the port to cause the body to conform to at least a portion of the tissue (step 405). The method includes a step of setting the shape-setting material such that the body continues to conform to the portion of the tissue upon removal of the negative pressure through the port (step 407).

The method 400 will now be described in greater detail with reference to the embodiments depicted in previous figures. The step 401 of providing a device including a body having an interior space and a port in fluid communication with the interior space can include, but is not limited to, providing a device 100 including a body 101 having an interior space 104 with a shape-setting material 105 included therein and a port 107 as described above with reference to Figs. 1A and 1B. The step 403 of applying the device to a tissue having an incision can include, but is not limited to, applying the device 100 to a tissue 150 having an incision 151 as described above with reference to Fig. 1B.

The step 405 of applying a negative pressure through the port to cause the body to conform to at least a portion of the tissue can include, but is not limited to, applying a negative pressure through the port 107 to cause the body 101 to conform to the tissue 150 as described above with reference to Fig. 1B. The step 407 of setting the shape-setting material such that the body continues to conform to the portion of the tissue upon removal of the negative pressure through the port can include, but is not limited to, setting the shape-setting material 105 such that the body 101 continues to conform to the tissue 150 upon removal of the negative pressure through the port 107.

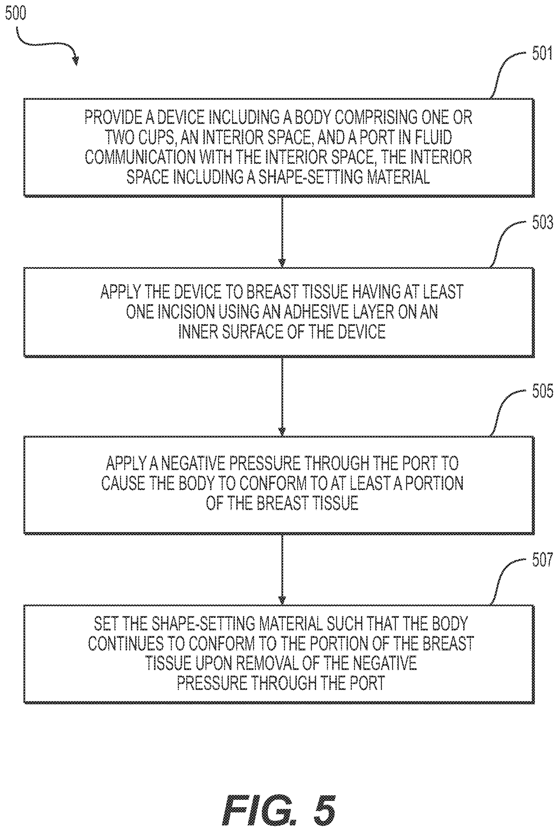

As shown in Fig. 5, a method 500 of treating breast tissue is provided in accordance with various embodiments of the present disclosure. The method 500 includes providing a device including a body comprising one or two cups, an interior space and a port in fluid communication with the interior space (step 501). The interior space includes a shape-setting material. The method 500 includes applying the device to breast tissue having at least one incision (step 503). The method 500 includes applying a negative pressure through the port to cause the body to conform to at least a portion of the breast tissue (step 505). The method 500 includes setting the shape-setting material such that the body continues to conform to the portion of the breast tissue upon removal of the negative pressure through the port (step 307).

The method 500 will now be described in greater detail with reference to the embodiments depicted in previous figures. The step 501 of providing a device including a body comprising one or two cups, an interior space and a port in fluid communication with the interior space can include, but is not limited to, providing a device 100 including one or two separated bodies 101 formed as cup-like portions 109, an interior space 104, and a port 107 as described with reference to Figs. 1 A and 1 B.

The step 503 of applying the device to breast tissue having at least one incision can include, but is not limited to, placing the device 100 over breast tissue 150 containing an incision 151. This step can be performed on a patient that has undergone a breast reconstruction or augmentation procedure that may include the placement of an implant. In some embodiments, the breast tissue may be in need of improved blood flow or re-vascularization to promote tissue healing and growth. The step 505 of applying a negative pressure through the port to cause the body to conform to at least a portion of the breast tissue can include, but is not limited to, connecting a vacuum to the port 107 to cause the cup-like portions 109 of the body 101 to closely conform to the shape of the breast tissue as described above with reference to Figs. 1A and 1B. When the shape-setting material hardens, the cups can retain their shape and, with the help of the adhesive layer 106, can resist decoupling from the tissue while providing support to the tissue.

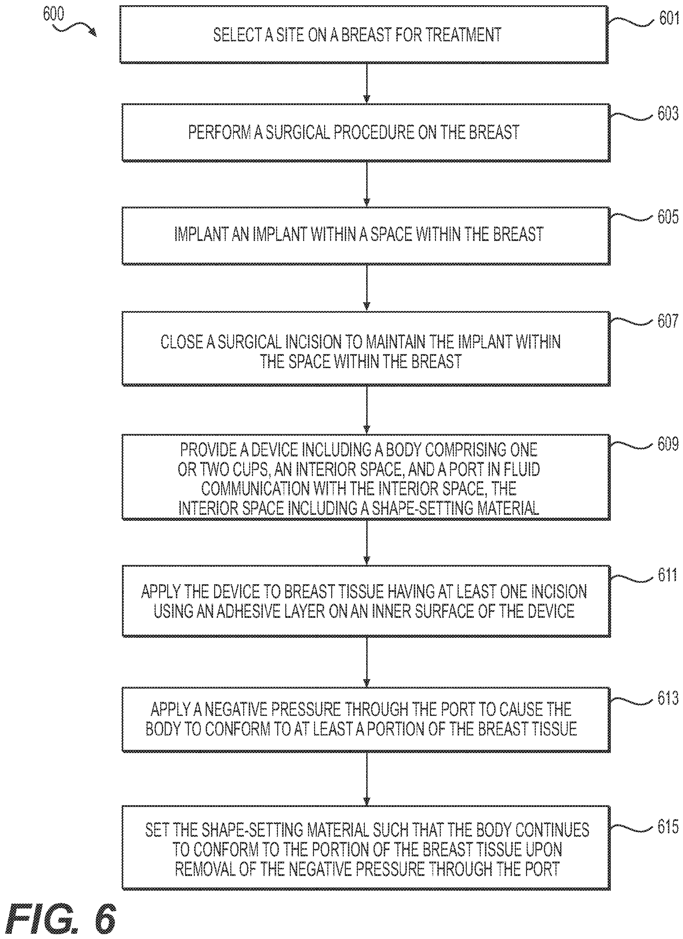

Fig. 6 is a flow chart showing steps for treating a breast including use of a device for post-surgery application in accordance with embodiments of the present disclosure. As shown, the process starts by selecting a site on a breast for treatment (Step 601). The selection will often be based on the need to remove tissue, for example, in surgical oncology, to remove breast tissue during mastectomy, skin-sparing mastectomy, lumpectomy, or any other procedure such as revision breast augmentation, breast augmentation, or mastopexy. It should be noted that as used herein, mastectomy or lumpectomy can include any variations on such procedures, such as radical mastectomy, modified radical mastectomy, or lumpectomy with sentinel node biopsy. In some cases, the site may be selected for augmentation or aesthetic procedures, and the procedure may include simply accessing the surgical site, and/or altering the site appearance.

After the site is selected, the surgical procedure (e.g., mastectomy, lumpectomy, surgical access) is performed on the breast (Step 603). In accordance with certain procedures, an implant 152 is implanted within a space within the breast (Step 605). The surgeon may then close the surgical incision (Step 607). For procedures that utilize an implant 152, the surgeon may close the surgical incision to maintain the implant within the space within the breast.

After completing the closure of the incision, a device is provided that includes a body having an interior space and a port in fluid communication with the interior space (Step 609). Further, the interior space includes a shape-setting material. The devices 100, 300 described above with reference to Figs. 1-3B are suitable as devices for use in this step. The device is applied to the breast near the surgical incision (Step 611). In some procedures, the device can surround the surgical incision. A

The surgeon can connect a source of negative pressure to the port of the device. The negative pressure is applied to cause the body to conform to at least a portion of the breast tissue (Step 613). As described previously, the shape-setting material can conform to the breast tissue when a negative pressure is applied. Finally, the shape-setting material is set such that the body continues to conform to the portion of the breast tissue upon removal of the negative pressure through the port (Step 615).

Other embodiments will be apparent to those skilled in the art from consideration of the specification and practice of this disclosure. It is intended that the specification and examples be considered as exemplary only, with the true scope and spirit of the disclosed devices and methods being indicated by the following claims.
Representative features are set out in the following clauses, which stand alone or may be combined, in any combination, with one or more features disclosed in the text and/or drawings of the specification.
1. A device for treating an anatomical region near a surgical site comprising: a body including an interior space; a shape-setting material disposed within the interior space; and a port disposed on the body and in fluid communication with the interior space, wherein application of negative pressure through the port causes the body to conform to at least a portion of an anatomical region near a surgical site, and wherein setting of the shape-setting material causes the body to continue to conform to the portion of the anatomical region upon removal of the negative pressure through the port.
2. The device of clause 1, wherein the body comprises a polymer.
3. The device of any of clauses 1-2, wherein an inner surface of the body includes an adhesive layer to adhere the device to the anatomical region.
4. The device of any of clauses 1-3, wherein the shape-setting material comprises a self-setting polymer.
5. The device of any of clauses 1-4, wherein the port is sealable to prevent fluid exchange.
6. The device of any of clauses 1-5, wherein the port comprises a one-way valve.
7. The device of any of clauses 1-6, wherein the anatomical region includes breast tissue.
8. The device of any of clauses 1-7, wherein accumulated fluids are removed through the port.
9. The device of any of clauses 1-8, further comprising an elastic bandage to maintain a position of the device relative to the anatomical region.
10. The device of any of clauses 1-9, wherein the anatomical region includes an incision.
11. A method of relieving stress on an anatomical region near a surgical site comprising: providing a device including a body having an interior space and a port in fluid communication with the interior space, the interior space including a shape-setting material; applying the device to an anatomical region near a surgical site; applying a negative pressure through the port to cause the body to conform to at least a portion of the anatomical region; and setting the shape-setting material such that the body continues to conform to the portion of the anatomical region upon removal of the negative pressure through the port.
12. The method of clause 11, wherein the body comprises a polymer.
13. The method of any of clauses 11-12, wherein an inner surface of the body includes an adhesive layer to adhere the device to the tissue.
14. The method of any of clauses 11-13, wherein the shape-setting material comprises a self-setting polymer.
15. The method of any of clauses 11-14, wherein the port is sealable to prevent fluid exchange.
16. The method of any of clauses 11-15, wherein the port comprises a one-way valve.
17. The method of any of clauses 11-16, wherein the anatomical region includes breast tissue.
18. The method of any of clauses 11-17, further comprising removing accumulated fluids through the port.
19. A method of treating breast tissue, comprising: providing a device including a body comprising one or two cups, an interior space, and a port in fluid communication with the interior space, the interior space including a shape-setting material; applying the device to breast tissue having at least one incision using an adhesive layer on an inner surface of the device; applying a negative pressure through the port to cause the body to conform to at least a portion of the breast tissue; setting the shape-setting material such that the body continues to conform to the portion of the breast tissue upon removal of the negative pressure through the port.
20. The method of clause 19, wherein the body comprises a polymer
21. The method of any of clauses 19-20, wherein the shape-setting material comprises a self-setting polymer.
22. The method of any of clauses 19-21, wherein the port is sealable to prevent fluid exchange.
23. The method of any of clauses 19-22, wherein the port comprises a one-way valve.
24. The method of any of clauses 19-23, wherein the anatomical region includes breast tissue.
25. The method of any of clauses 19-24, further comprising removing accumulated fluids through the port.
26. A method for treating a breast, comprising: performing a surgical procedure on a breast; implanting an implant within a space within the breast; closing a surgical incision to maintain the implant within the space within the breast; providing a device including a body having an interior space and a port in fluid communication with the interior space, the interior space including a shape-setting material; applying the device to the breast near the surgical incision; applying a negative pressure through the port to cause the body to conform to at least a portion of the breast; and setting the shape-setting material such that the body continues to conform to the breast upon removal of the negative pressure through the port.

## Claims

1. A method of setting a device (100), comprising:
providing the device, the device including a body (101) comprising one or two cups (109), an interior space (104), and a port (107) in fluid communication with the interior space (104), the interior space including a shape-setting material (105);
applying the device to breast tissue (150) having at least one incision (151) using an adhesive layer (106) on an inner surface of the device;
applying a negative pressure through the port to cause the body to conform to at least a portion of the breast tissue; and
setting the shape-setting material such that the body continues to conform to the portion of the breast tissue upon removal of the negative pressure through the port.

2. The method of claim 1, wherein the body comprises a polymer.

3. The method of any of claims 1-2, wherein the shape-setting material comprises a self-setting polymer.

4. The method of any of claims 1-2, wherein setting the shape-setting material includes thermosetting a polymer.

5. The method of any of claims 1-2, wherein setting the shape-setting material includes curing liquid silicone rubber using ultraviolet light.

6. The method of any of claims 1-5, further comprising sealing the port to prevent fluid exchange.

7. The method of any of claims 1-6, wherein the port comprises a one-way valve.

8. The method of any of claims 1-7, further comprising removing accumulated fluids through the port.

9. The method of claim 8, wherein removing accumulated fluids includes drawing fluid away from the incision using a wicking layer.

10. The method of any of claims 1-9, further comprising applying a drape connected to an outer layer of the device to surrounding tissue to maintain a position of the device relative to the breast tissue.

11. The method of claim 10, wherein the drape is an elastic bandage.

12. The method of claim 10, wherein the drape is an adhesive film.

13. The method of any one of claims 1-12, wherein the device comprises a heat-shielding or heat-reflecting material that thermally isolates the breast tissue from the shape-setting material.

14. The method of any one of claims 1-13, wherein the interior space (104) includes a first interior space (104a) that includes the shape-setting material (105) and a second interior space (104b) that is in fluid communication with the port (107).
